# DEMANDE DE BREVET EUROPEEN

(11) **EP 1 543 818 A1**
(43) Date de publication de la demande: **22.06.2005**
(21) Numéro de dépôt: 04292685.7
(22) Date de dépôt: 12.11.2004
(51) Int. Cl.: A61K 7/075

(54) **Composition cosmétique comprenant un agent cationique, un polymère comprenant un hétéroatome et une huile et procédé de traitement cosmétique**

(30) Priorité: 19.12.2003 FR 0351145
(71) Demandeur: L'OREAL, 75008 Paris (FR)
(72) Inventeur: Parris, Eric, 92110 Clichy (FR)
(74) Mandataire: Le Blainvaux Bellegarde, Françoise

(57) **Abrégé**

La présente invention est relative à une composition cosmétique comprenant, dans un milieu cosmétiquement acceptable, au moins au moins un agent cationique, au moins une huile et au moins un polymère à hétéroatomes et chaînes grasses et à un procédé de traitement cosmétique des matières kératiniques en particulier les cheveux.

Ces compositions possèdent un effet conditionneur amélioré et rémanent.

## Description

La présente invention est relative à une composition cosmétique notamment de conditionnement des cheveux comprenant au moins un agent cationique, au moins un polymère comportant un hétéroatome associé à au moins une huile et à un procédé de traitement cosmétique des matières kératiniques en particulier les cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.
Dans le domaine de la cosmétique, on cherche notamment à améliorer le conditionnement des cheveux. Par conditionnement, on entend des propriétés de démêlage facile, de brillance, de douceur au toucher et de glissant et de lissage visuel et au toucher.

On a déjà proposé pour le traitement des matières kératiniques et en particulier des cheveux, des compositions cosmétiques contenant des tensioactifs cationiques.

De telles compositions présentent cependant des inconvénients tels que des problèmes de rinçabilité, des problèmes de stabilité, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques, de silicones cationiques ou de tensioactifs cationiques pour faciliter le démêlage des cheveux et pour leur communiquer douceur et souplesse. L'usage des polymères cationiques ou des cations dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

En résumé, il s'avère que les compositions cosmétiques actuelles de conditionnement, ne donnent pas complètement satisfaction. Ainsi, on cherche à obtenir des compositions cosmétiques ayant des propriétés de conditionnement améliorées en particulier un toucher plus lisse.

L'utilisation de polymère à chaîne grasse terminale et à motif récurrent contenant au moins un hétéroatome est connue en cosmétique, et plus particulièrement dans le domaine du maquillage comme cela est décrit dans les demandes de brevet FR2817743. Des compositions cosmétiques dont la phase grasse est gélifiée par de tels polymères ont été décrites la demande FR 2796270 qui décrit plus particulièrement des compositions solides de rouge à lèvre sous la forme d'un stick .

Le brevet US 5 783 657 illustre certains types des polymères qui peuvent entrer dans la composition des formules de l'invention.
Néanmoins, ces documents ne décrivent pas de compositions cosmétiques contenant un agent cationique, en particulier un tensioactif cationique.

La demanderesse a maintenant découvert que l'association d'au moins un agent cationique, d'au moins un polymère à chaîne grasse terminale ou pendante et à motif récurrent contenant au moins un hétéroatome et d'au moins une huile, notamment dans des milieux non détergents ayant une concentration faible ou nulle en agents tensio-actifs lavants permet de remédier à ces inconvénients.

Les cheveux traités avec cette composition sont lisses, se démêlent facilement sont brillants, souples, individualisés et ont un toucher doux et sans résidus. Les cheveux ont un aspect naturel et non chargé.

Sans être tenu par une quelconque théorie, il semblerait que dans ces conditions, on augmente significativement le dépôt d'huile sur les cheveux, d'où une efficacité accrue. Cette amélioration se fait cependant sans avoir un toucher chargé gras, ce qui est le cas habituellement lorsqu'on augmente la quantité d'huile.

Par ailleurs, cet effet de conditionnement peut être rémanent au rinçage .

Ainsi, selon la présente invention, il est maintenant proposé de nouvelles compositions cosmétiques notamment non lavantes , comprenant, dans un milieu aqueux cosmétiquement acceptable, au moins un agent cationique, de 0,005 à 5% en poids d'au moins une huile, au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés et de 75 à 98 % en poids d'eau par rapport au poids total de la composition.

L'invention a également pour objet une composition cosmétique, comprenant, dans un milieu aqueux cosmétiquement acceptable, au moins un agent cationique choisi parmi les tensioactifs cationiques, au moins une huile et au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés.

Un autre objet de l'invention consiste en un procédé de traitement cosmétique des matières kératiniques, en particulier les cheveux mettant en oeuvre la composition susvisée.

L'invention a encore pour objet l'utilisation de ladite composition comme après-shampoing.

D'autres objets, caractéristiques, aspects et avantages de l'invention apparaîtront encore plus clairement à la lecture de la description et des divers exemples qui suivent.

Selon un mode particulier de l'invention, l'huile est, de préférence, pré-épaissie par le polymère ci-dessus, c'est à dire qu'on mélange l'huile et le polymère avant l'introduction dans la composition.

Le rapport pondéral huile(s)/polymère(s) de l'invention est de préférence supérieur ou égal à 50/50, mieux encore supérieur ou égal à 60/40, plus préférentiellement compris entre 60/40 et 99/1 et encore plus préférentiellement compris entre 80/20 et 99/1.

Cette huile de préférence pré-épaissie est dispersée sous forme de particules dans la composition aqueuse. Les particules d'huile présentent de préférence une taille primaire moyenne en nombre comprise entre 1 et 100 µm, mieux encore entre 5 et 30 µm, et plus particulièrement de 10 à 20 µm .

Au sens de la présente invention, on entend par "taille primaire de particule", la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Le polymère structurant de la composition de l'invention est un solide non déformable à température ambiante (25°C) et pression atmosphérique (760 mm de Hg). Il est insoluble dans l'eau ou la phase aqueuse ; il est capable de structurer l'huile. En particulier, le polymère structurant ne cristallise pas et la structuration de la phase grasse liquide est due à des interactions hydrogène entre deux molécules de polymère et/ou entre les molécules du polymère et les molécules de la phase grasse liquide. De préférence, le polymère structurant n'a pas de groupe ionique.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substituées au moins partiellement par des atomes de fluor.

Selon l'invention, ces chaînes peuvent être liées directement au squelette polymérique ou via une fonction ester ou un groupement perfluoré.

Par "polymère", on entend au sens de l'invention un composé ayant au moins 2 motifs de répétition, et de préférence au moins 3 motifs de répétition, qui sont identiques.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes avantageusement non pendants et se trouvant dans le squelette polymérique. Ces hétéroatomes sont choisis parmi les atomes d'azote, de soufre, de phosphore et leurs associations, associés éventuellement à un ou plusieurs atomes d'oxygène. De préférence, les motifs comportent au moins un atome d'azote en particulier non pendant. Ces motifs comportent, en outre, avantageusement, un groupe carbonyle.

Les motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide. Avantageusement, les chaînes pendantes sont liées directement à l'un au moins des hétéroatomes du squelette polymérique. Selon un mode de réalisation, le premier polymère comprend un squelette polyamide. En outre, les chaînes terminales sont liées au squelette polymérique, via un groupe de liaison qui peut être un groupe éther, amine, urée, uréthane, thioether, thioester, thiourée, thiouréthane ou une liaison simple.

Entre les motifs hydrocarbonés, le polymère peut comprendre des motifs siliconés ou des motifs oxyalkylénés.

En outre, le polymère de la composition de l'invention comprend avantageusement un nombre total de chaînes grasses qui représente de 40 à 98 % du nombre total des motifs à hétéroatomes et des chaînes grasses, et mieux de 50 à 95 %. La nature et la proportion des motifs à hétéroatome est fonction de la nature de la phase grasse liquide et est en particulier similaire à la nature (polaire ou non) de la phase grasse liquide. Ainsi, plus les motifs à hétéroatome sont polaires et en proportion élevée dans le polymère, ce qui correspond à la présence de plusieurs hétéroatomes, plus le polymère a de l'affinité avec les huiles polaires. En revanche, plus les motifs à hétéroatome sont peu polaires voire apolaires ou en proportion faible, plus le polymère a de l'affinité avec les huiles apolaires.

Le polymère est de préférence un polyamide de masse moléculaire moyenne en poids inférieure à 1 000 000, comportant a) un squelette polymérique, ayant des motifs répétitifs amide, et b) éventuellement au moins une chaîne grasse pendante et/ou au moins une chaîne grasse terminale éventuellement fonctionnalisées, ayant de 6 à 120 atomes de carbone et étant liées à ces motifs amide.

De préférence, les chaînes grasses pendantes sont liées à l'un au moins des atomes d'azote des motifs amide du polymère.

En particulier, les chaînes grasses de ce polyamide représentent de 40 à 98 % du nombre total des motifs à hétéroatomes et des chaînes grasses du polymère, et mieux de 50 à 95 %.

Avantageusement, le polymère structurant et en particulier le polyamide, de la composition selon l'invention présente une masse moléculaire moyenne en poids inférieure à 1 000 000 et mieux à 500 000. De préférence, cette masse moléculaire est inférieure ou égale à 100 000 (notamment allant de 1000 à 100 000), en particulier inférieure ou égale à 50 000 (notamment allant de 1000 à 50 000), et plus particulièrement allant de 1000 à 30 000, de préférence de 2000 à 20 000, et mieux de 2000 à 10 000.

Comme polymères structurant préférés utilisables dans l'invention, on peut citer les polyamides de préférence ramifiés par des chaînes grasses pendantes et/ou des chaînes grasses terminales contenant de 6 à 120 atomes de carbone, en particulier ayant de 12 à 120 atomes de carbone et notamment de 12 à 68 atomes de carbone, les chaînes grasses terminales étant liées au squelette polyamide par des groupes de liaison notamment ester.

Ces polymères sont de préférence des polymères résultant d'une polycondensation entre un diacide carboxylique à au moins 32 atomes de carbone (ayant notamment de 32 à 44 atomes de carbone) et une diamine ayant au moins 2 atomes de carbone (notamment de 2 à 36 atomes de carbone). Le diacide est de préférence un dimère d'acide gras ayant au moins 16 atomes de carbone comme l'acide oléique, linoléique ou linolénique. La diamine est de préférence l'éthylène diamine, l'hexylène diamine, l'hexaméthylène diamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5783657 de la société Union Camp. Chacun de ces polymères satisfait notamment à la formule (I) suivante : 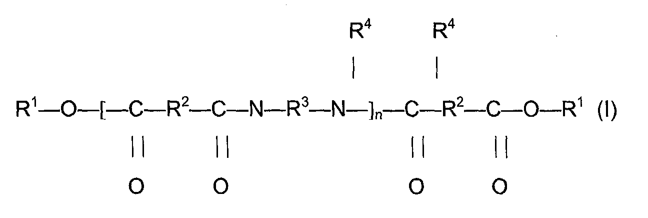 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone par exemple de 4 à 24 atomes de carbone; R² représente à chaque occurrence indépendamment un groupe hydrocarboné en C₄ à C₄₂ à condition que 50 % des groupes R² représentent un groupe hydrocarboné en C₃₀ à C₄₂; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 10 par exemple, de 1 à 5 et mieux supérieur à 2. De préférence, R¹ est un groupe alkyle en C₁₂ à C₂₂ et de préférence en C₁₆ à C₂₂. Avantageusement, R² peut être un groupe hydrocarboné (alkylène) en C₁₀ à C₄₂. De préférence, 50 % au moins et mieux 75 % des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂.

Les groupes hydrocarbonés peuvent être des groupes linéaires, cycliques ou ramifiés, saturés ou insaturés. Par ailleurs, les groupes alkyle et alkényle peuvent être des groupes linéaires ou ramifiés.

Selon l'invention, la structuration (ou épaississement) de la phase grasse liquide est obtenue à l'aide d'un ou plusieurs polymères de formule (I). En général, les polymères de formule (I) se présentent sous forme de mélanges de polymères, ces mélanges pouvant en outre contenir un produit de synthèse correspondant à un composé de formule I) où n vaut 0, c'est-à-dire un diester.

A titre d'exemple de polymères structurant utilisables dans la composition selon l'invention, on peut citer les produits commerciaux fabriqués ou vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en C₃₆ condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelé aussi alcool cétylstéarylique).

Comme polymère structurant utilisable dans l'invention, on peut encore citer les résines polyamides résultant de la condensation d'un acide di-carboxylique aliphatique et d'un diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides fabriqués ou vendus par la société Arizona Chemical sous les références Uni-Rez® (2658, 2931, 2970, 2621, 2613, 2624, 2665, 1554, 2623, 2662) et le produit vendu sous la référence Macromelt 6212 de la société Henkel. Pour plus d'information sur ces polyamides, on peut se référer au document US-A-5500209.

Il est aussi possible d'utiliser des résines de polyamides issues de légumes comme celles décrites dans les brevets US-A-5783657 et US-A-5998570.

Les polymères structurant de la composition de l'invention ont avantageusement une température de ramollissement supérieure à 65°C et mieux supérieure à 70°C et pouvant aller jusqu'à 190°C. De préférence, ils présentent une température de ramollissement inférieur à 150°C, par exemple allant de 70 à 140°C et mieux allant de 80 à 130°C et mieux encore de 80 à 105°C. Ces polymères sont en particulier des polymères non cireux. Le bas point de fusion des polymères structurant de l'invention facilite leur mise en oeuvre et limite la dégradation de la phase grasse liquide, contrairement à des polymères ou composés de point de ramollissement plus élevé.

De préférence, les polymères de la composition selon l'invention sont ceux répondant à la formule (I). Ces polymères présentent du fait de leur (s) chaîne (s) grasse (s), une bonne solubilité dans les huiles et donc conduisent à des compositions macroscopiquement homogènes même avec un taux élevé (au moins 25%) de polymère, contrairement à des polymères exempts de chaîne grasse.

Dans toute la description, les valeurs de température de ramollissement ou de fusion peuvent être déterminées par la méthode D.S.C ("Differential Scanning Calorimetry") ; la température de ramollissement ou de fusion correspond alors au pic de fusion et la montée en température est de 5 ou 10°C/min.

L'épaississement de la phase grasse est modulable selon la nature du ou des polymères et leurs concentrations et peut être telle que l'on obtienne de préférence une viscosité allant de 1000 à 250.000 cps et de préférence de 10.000 à 50.000 cps, à 25°C mesurée avec un appareil RHEOMAT 180 avec un taux de cisaillement de 100s⁻¹.

Le polymère tel que défini ci-dessus est de préférence présent en une quantité comprise entre 0,005 et 20 % en poids, mieux encore en une quantité comprise entre 0,05 et 10 % en poids, et encore plus préférentiellement en une quantité comprise entre 0,1 et 5 % en poids par rapport au poids de la composition.

Par huile, au sens de la demande, on entend un corps gras liquide insoluble dans l'eau à température ambiante (25°C) et pression atmosphérique (760 mm de Hg). La phase huileuse peut être composée d'une ou plusieurs huiles, compatibles entre elles.

Par insoluble dans l'eau, on entend au sens de la présente invention, une substance qui présente une solubilité dan l'eau pure inférieure à 1 % à 25 °C et sous pression atmosphérique.

Les huiles utilisées dans la présente invention présente une viscosité dynamique à 25 °C, inférieure à 1 Pa.s (1000 cps), de préférence comprise entre 10⁻³ et 0,1 Pa.s (1 et 100 cps). La viscosité dynamique est mesurée à 25 °C avec un taux de cisaillement de 100 s⁻¹, par exemple, avec l'appareil référencé RM 180 Rheomat de la société METTLER.

Les huiles pouvant être utilisées dans la présente invention sont choisies de préférence, parmi les huiles végétales, les huiles minérales, les huiles de synthèse, et les esters d'acide gras.

Parmi les huiles végétales pouvant être utilisées dans la présente invention, on peut notamment citer l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot et l'huile de calophyllum.

Des exemples d'huiles minérales sont l'huile de paraffine et l'huile de vaseline.
Les huiles de synthèse peuvent notamment être choisies parmi les polydécènes, le squalane, les poly(alpha-oléfines) comme l'isododécane ou l'isohexadécane, les huiles végétales transestérifiées et les huiles fluorées.

On peut également utiliser des esters d'acide gras, tels que par exemple, les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle.

Les huiles particulièrement préférées dans le cadre de la présente invention sont l'huile d'avocat, l'huile de ricin, l'huile d'olive, le polydécène hydrogéné, le myristate d'isopropyle, l'isononanoate d'isononyle, une paraffine liquide

La ou les huile(s) telle(s) que définie(s) ci-dessus est ou sont de préférence présente(s) en une quantité comprise allant de 0,005 à 30 % en poids, encore plus préférentiellement en une quantité allant de 0,01 à 15 % en poids et plus particulièrement de 0,01 à 5% en poids par rapport au poids de la composition.

La ou le(s) huile(s) est ou sont présente(s) de préférence en une quantité comprise allant de 0,01 à 3 % en poids, de préférence de 0,01 à 1 % en poids par rapport au poids total de la composition.

Les agents cationiques sont de préférence choisis parmi les tensioactifs cationiques et les polymères cationiques et/ou leur mélanges et plus particulièrement parmi les tensioactifs cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 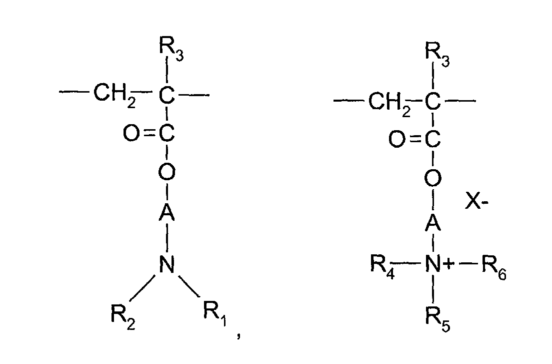 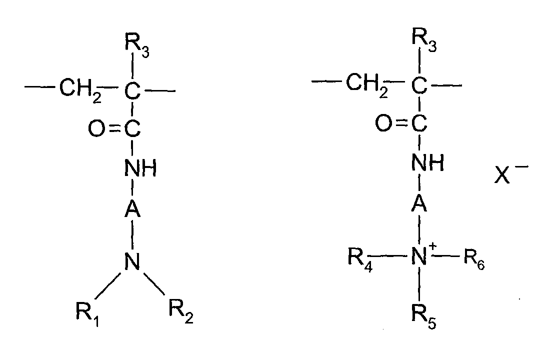 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymères vinylpyrrolidone / méthacrylamidopropyl dimethylamine commercialisés notamment sous la dénomination STYLEZE CC 10 par ISP.
   - et les copolymères vinylpyrrolidone / méthacrylamide de diméthylaminopropyle quaternisé tel que le produit vendu sous la dénomination "GAFQUAT HS 100" par la société ISP.
(2)Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyoxyléthyl triméthylammonium, de méthacrylamidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US 3 589 578 et 4 031 307 en particulier les gommes de guar comprenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société RHODIA.
(3)les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4)les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5)les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6)les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7)les cyclopolymères d'alkyl diallyl amine ou de dialkyl diallyl ammonium tels que les homopolymères ou copolymères comportant comme constituant principal de la chaîne des motifs répondant aux formules (II) ou (III) : 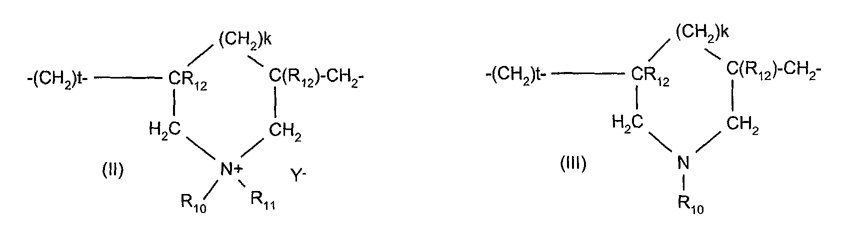 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses moléculaires moyenne en poids) et les copolymères de chlorure de diallyldiméthylammonium et d'acrylamide commercialisés sous la dénomination "MERQUAT 550".
(8)les polymères de diammonium quaternaire comprenant des motifs récurrents répondant à la formule : 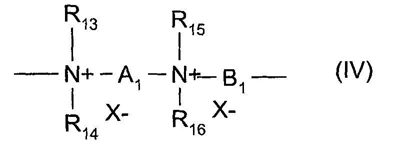 formule (IV) dans laquelle :
   R₁₃, R₁₄, R₁₅ et R₁₆, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;

   A1 et B1 représentent des groupements polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;
   n, identique ou différent, représente un nombre entier allant de 2 à 20, de préférence de 2 à 6,
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 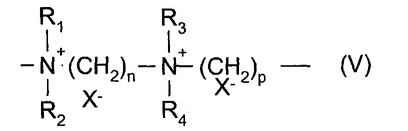 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (V) particulièrement préféré est celui pour lequel R₁, R₂, R₃ et R₄, représentent un radical méthyle et n = 3, p = 6 et X = Cl, dénommé Hexadimethrine chloride selon la nomenclature INCl (CTFA).
(9)les polymères de polyammonium quaternaires comprenant des motifs de formule (VI): 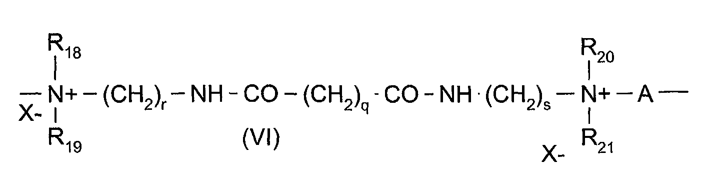 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
      où p est égal à 0 ou à un nombre entier compris entre 1 et 6, sous réserve que R₁₈, R₁₉, R₂₀ et R₂₁ ne représentent pas simultanément un atome d'hydrogène,
      r et s, identiques ou différents, sont des nombres entiers compris entre 1 et 6,
      q est égal à 0 ou à un nombre entier compris entre 1 et 34,
      X⁻ désigne un anion tel qu'un halogènure,
      A désigne un radical d'un dihalogénure ou représente de préférence -CH₂-CH₂-O-CH₂-CH₂-.
      De tels composés sont notamment décrits dans la demande de brevet EP-A-122 324.
      On peut par exemple citer parmi ceux-ci, les produits "Mirapol® A 15", "Mirapol® AD1", "Mirapol® AZ1" et "Mirapol® 175" vendus par la société Miranol.
(10)Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de « POLYETHYLENEGLYCOL (15) TALLOW POLYAMINE » dans le dictionnaire CTFA.
(12) Les polymères de préférence réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination « JR 400 » par la Société AMERCHOL, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,001 à 20%, de préférence de 0,05 à 10% et plus particulièrement de 0,1 à 5 % en poids par rapport au poids total de la composition.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaire, on peut notamment citer, par exemple :
- ceux qui présentent la formule générale (V) suivante : 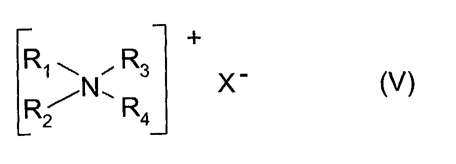
dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (VI) suivante : 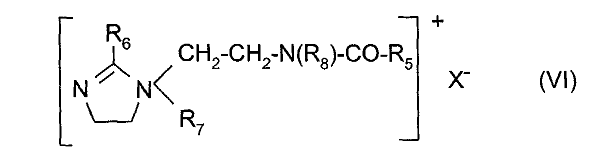
dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT®" W 75, W90, W75PG, W75HPG par la société WITCO,
- les sels de diammonium quaternaire de formule (VII) : 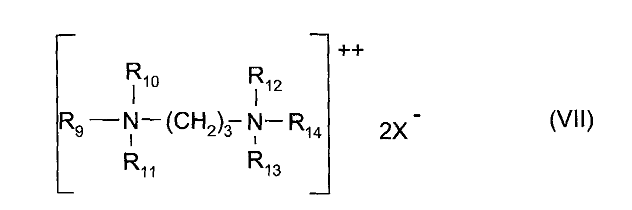
dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire contenant au moins une fonction ester, tels que ceux de formule (VIII) suivante : 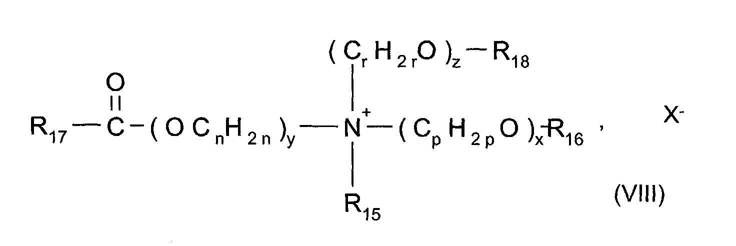
dans laquelle :
R₁₅ est choisi parmi les radicaux alkyles en C₁-C₆ et les radicaux hydroxyalkyles ou dihydroxyalkyles en C₁-C₆ ;
R₁₆ est choisi parmi :
   - le radical 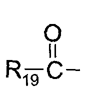
   - les radicaux R₂₀ hydrocarbonés en C₁-C₂₂, linéaires ou ramifiés, saturés ou insaturés,
   - l'atome d'hydrogène,
R₁₇ est choisi parmi :
   - le radical 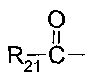
   - les radicaux R₂₂ hydrocarbonés en C₁-C₆, linéaires ou ramifiés, saturés ou insaturés,
   - l'atome d'hydrogène,
R₁₇, R₁₉ et R₂₁, identiques ou différents, sont choisis parmi les radicaux hydrocarbonés en C₇-C₂₁, linéaires ou ramifiés, saturés ou insaturés ;
r, n et p, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés, et plus particulièrement linéaires.

De préférence, R₁₅ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou
différents, valent 0 ou 1.

Avantageusement, y est égal à 1.

De préférence, r, n et p, identiques ou différents, valent 2 ou 3, et encore plus particulièrement sont égaux à 2.

L'anion X⁻ est de préférence un halogénure (chlorure, bromure ou iodure) ou un alkyl(C1-C4)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1 ;
- r, n et pt sont égaux à 2 ;
- R₁₆ est choisi parmi :
- le radical 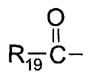
- les radicaux méthyle, éthyle ou hydrocarbonés en C₁₄-C₂₂,
- l'atome d'hydrogène ;
- R₁₈ est choisi parmi :
- le radical 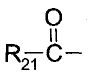
- l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.
Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société COGNIS, STEPANQUAT® par la société STEPAN, NOXAMIUM® par la société CECA, REWOQUAT® WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire mentionnés ci-dessus, on préfère utiliser ceux répondant à la formule (V). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le chlorure de béhénylamidopropyl 2,3-dihydroxypropyl diméthyl ammonium et le chlorure de palmitylamidopropyltriméthylammonium.

La composition selon l'invention contient de préférence le ou les tensioactifs cationiques en une quantité allant de 0,05 à 10 % en poids, de préférence de 0,1 à 8 % en poids par rapport au poids total de la composition et plus particulièrement de 0,2 à 6% en poids et encore plus préférentiellement de 0,3 à 3% en poids.

La composition selon l'invention peut contenir éventuellement d'autres tensioactifs que les tensioactifs cationiques, notamment non ioniques ou amphotères.

Les tensioactifs additionnels sont généralement présents une quantité comprise entre 0,1% et 10% en poids environ, de préférence entre 0,5% et 8% et encore plus préférentiellement entre 1% et 5%, par rapport au poids total de la composition.

La composition selon l'invention contient de préférence au moins un tensioactif choisi parmi les tensioactifs non ioniques.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés, ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

Les compositions non lavantes (non détergentes) comprennent de préférence moins de 4% en poids de tensioactifs détergents anioniques et plus particulièrement moins de 1% en poids par rapport au poids total de la composition.

La composition selon l'invention peut comprendre en outre au moins un agent conditionneur.

Dans le cadre de la présente demande, on entend par agent conditionneur tout agent ayant pour fonction l'amélioration des propriétés cosmétiques des matières kératiniques telles que les cheveux, en particulier la douceur, le lissage, le démêlage, le toucher, l'électricité statique.

Les agents conditionneurs peuvent être solubles ou insolubles dans l'eau.

Les agents conditionneurs sont notamment choisis parmi les cires naturelles ou synthétiques, les composés de type céramide, les esters d'acides carboxyliques, les silicones, parmi les polymères anioniques, les polymères non ioniques, les polymères cationiques, les polymères amphotères, les protéines cationiques, les hydrolysats de protéines cationiques, habituellement utilisés dans les compositions cosmétiques ou dermatologiques ainsi que les mélanges de ces différents composés.
Les agents conditionneurs insolubles conformément à l'invention peuvent être solides, liquides ou pâteux à température ambiante (25°C) et à pression atmosphérique, ils peuvent notamment se présenter sous forme d'huiles, de cires, de résines ou de gommes.

Les agents conditionneurs insolubles sont notamment dispersés dans les compositions sous forme de particules ayant généralement une taille moyenne en nombre comprise entre 2 nanomètres et 100 microns, de préférence entre 30 nanomètres et 20 microns (mesurée avec un granulomètre).

Les agents conditionneurs sont contenus de préférence dans la composition selon l'invention en une quantité allant de 0,01 % à 20 % en poids, mieux encore allant de 0,05 % à 10 % en poids et plus particulièrement allant de 0,1 % à 5 % en poids par rapport au poids total de la composition.

Le milieu cosmétiquement acceptable est de préférence aqueux et peut être comprendre de l'eau ou un mélange d'eau et d'un solvant cosmétiquement acceptable tel qu'un alcool inférieur en C₁-C₄, par exemple l'éthanol, l'isopropanol, le tertio-butanol, le n-butanol ; les polyols comme le propylèneglycol, la glycérine ; les éthers de polyols ; les alcanes en C₅-C₁₀ ; et leurs mélanges.. Les solvants sont de préférence choisis parmi la glycérine et le propylène glycol.

Le milieu cosmétiquement acceptable notamment aqueux représente de 30 à 98% en poids par rapport au poids total de la composition et de préférence de 80 à 98% en poids.

De préférence, la composition comprend de 75 à 99 % en poids d'eau par rapport au poids total de la composition et de préférence de 80 à 98% en poids.

Les solvants sont de préférence présents dans des concentrations allant de 0,5 à 30% en poids par rapport au poids total de la composition.

Le pH des compositions de l'invention est compris entre 2 et 8, de préférence entre 3 et 7,5.

Les compositions selon l'invention peuvent également contenir des additifs classiques bien connus dans la technique, tels que des polymères anioniques, non ioniques ou amphotères, des épaississants non polymériques comme des acides ou des électrolytes, des opacifiants, des nacrants, des vitamines, des provitamines telles que le panthénol, des alcools gras, des cires telles que les cires végétales, des céramides naturels ou synthétiques, des parfums, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions de l'invention peuvent se présenter sous forme d'après-shampooing à rincer ou non, de compositions pour permanente, défrisage, coloration ou décoloration, ou encore sous forme de compositions éventuellement à rincer, à appliquer avant ou après une coloration, une décoloration, une permanente ou un défrisage ou encore entre les deux étapes d'une permanente ou d'un défrisage.

Elles peuvent être utilisées, par exemple, comme après-shampoings, soins, masques de soin profond, lotions ou crèmes de traitement du cuir chevelu. Ces compositions peuvent être rincées ou non rincées.

Selon un mode de réalisation préféré de l'invention, la composition peut être utilisée comme après-shampoing, en particulier sur des cheveux fins. Cet après-shampooing peut être rincé ou non rincé et de préférence rincé.

Les compositions cosmétiques selon l'invention peuvent se présenter sous forme de gel, de lait, de crème, d'émulsion, de lotion fluides ou épaissies ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

La présente invention concerne également un procédé de traitement cosmétique des matières kératiniques telles que par exemple la peau ou les cheveux qui consiste à appliquer une quantité efficace d'une composition cosmétique telle que décrite ci-dessus, sur les matières kératiniques, à effectuer un éventuel rinçage après un éventuel temps de pose.
Le rinçage s'effectue par exemple avec de l'eau.

Ainsi, ce procédé selon l'invention permet le maintien de la coiffure, le traitement, le conditionnement, le soin des cheveux ou de toute autre matière kératinique.

Les exemples suivants illustrent la présente invention et ne doivent être considérés en aucune manière comme limitant l'invention.

### EXEMPLES 1 ET 2

On a préparé les compositions suivantes d'après-shampooing à rincer :

| Composition | 1 | 2 |
|---|---|---|
| Inuline (Raftiline HP- ORAFTI) | 15 g | --- |
| Polyquaternium-37 (Salcare SC 95-CIBA) | 0.5 g | --- |
| Alcool Cétylstéarylique | --- | 2 g |
| Cetyl esters (Spermwax vegetal-ROBECO) | --- | 0.25 g |
| Behentrimonium Chloride (Genamin KDMP - CLARIANT) | --- | 1.4 g |
| Mélange (95/5 en poids) de Copolymère d'un diacide en C₃₆ condensé sur l'éthylène diamine (UNICLEAR 100VG de ARIZONA CHEMICAL) et de Polydécène hydrogéné (CERAFLOW E de SHAMROCK) | 0.5 g | 1 g |
| Conservateur | qs | qs |
| Agent de pH qs | pH = 7 | pH = 3.5 |
| Eau | Qsp 100 g | Qsp 100 g |

On applique ces compositions sur des cheveux sensibilisés. Après 3 minutes de pose, on rince les cheveux. Ces cheveux se démêlent facilement et sont très lisses.

## Revendications

1. Composition cosmétique, comprenant, dans un milieu aqueux cosmétiquement acceptable, au moins un agent cationique, de 0,005 à 5% en poids d'au moins une huile, au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés et de 75 à 98 % en poids d'eau par rapport au poids total de la composition.

2. Composition cosmétique, comprenant, dans un milieu aqueux cosmétiquement acceptable, au moins un agent cationique choisi parmi les tensioactifs cationiques, au moins une huile et au moins un polymère de masse moléculaire moyenne en poids inférieure ou égale à 1 000 000, comportant a) un squelette polymérique, ayant des motifs de répétition hydrocarbonés pourvus d'au moins un hétéroatome, et b) au moins une chaîne grasse pendante et/ou au moins une chaîne grasse terminale éventuellement fonctionnalisées, ayant de 6 à 120 atomes de carbone et étant liées à ces motifs hydrocarbonés.

3. Composition selon la revendication 1 ou 2, **caractérisée par le fait que** la masse moléculaire moyenne du premier polymère est inférieure ou égale à 500 000 et mieux inférieure ou égale à 100 000.

4. Composition selon l'une quelconque des revendications précédentes, **caractérisée par le fait que** les motifs à hétéroatome du polymère comportent au moins un atome d'azote.

5. Composition selon l'une des revendications précédentes, **caractérisée par le fait que** les motifs à hétéroatome sont des groupes amides.

6. Composition selon l'une des revendications précédentes, **caractérisée par le fait que** les chaînes grasses représentent de 40 à 98 % du nombre total des motifs à hétéroatome et des chaînes grasses.

7. Composition selon l'une des revendications précédentes, **caractérisée par le fait que** les chaînes grasses représentent de 50 à 95 % du nombre total des motifs à hétéroatome et des chaînes grasses.

8. Composition selon l'une des revendications précédentes, **caractérisée par le fait que** les chaînes grasses pendantes sont liées directement à l'un au moins desdits hétéroatomes.

9. Composition selon l'une des revendications 4 à 8, **caractérisée par le fait que** les chaînes grasses pendantes sont liées directement à l'un au moins des atomes d'azote des motifs amide.

10. Composition selon l'une des revendications précédentes, **caractérisée par le fait que** la masse moléculaire moyenne en poids va de 1 000 à 30 000, et mieux de 2 000 à 10 000.

11. Composition selon l'une des revendications précédentes, **caractérisée par le fait que** les chaînes grasses terminales sont liées au squelette par des groupes de liaison.

12. Composition selon la revendication 10, **caractérisée par le fait que** les groupes de liaison sont des groupes ester.

13. Composition selon l'une des revendications précédentes, **caractérisée par le fait que** les chaînes grasses ont de 12 à 68 atomes de carbone.

14. Composition selon l'une des revendications précédentes, **caractérisée par le fait que** le polymère est choisi parmi les polymères de formule (I) suivante et leurs mélanges : 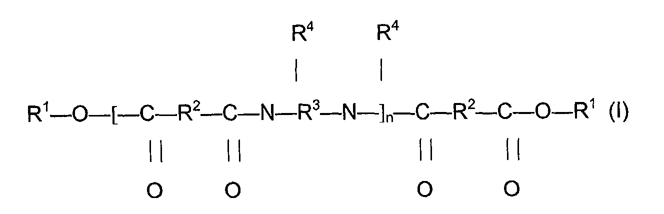 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

15. Composition selon la revendication précédente, **caractérisée par le fait que** R¹ est un groupe alkyle en C₁₂ à C₂₂.

16. Composition selon l'une des revendications 14 ou 15, **caractérisée par le fait que** R² sont des groupes ayant de 30 à 42 atomes de carbone.

17. Composition selon l'une quelconque des revendications précédentes, **caractérisée en ce que** le polymère est présent en une quantité comprise entre 0,005 et 20 % en poids, de préférence entre 0,05 et 10 % en poids par rapport au poids total de la composition.

18. Composition selon l'une quelconque des revendications précédentes, **caractérisée en ce que** l'huile est choisie parmi les huiles végétales, les huiles minérales, les huiles de synthèse, et les esters d'acides gras.

19. Composition selon la revendication 18, **caractérisée en ce que** l'huile est choisie parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile de paraffine et l'huile de vaseline ; les polydécènes, le squalane, les poly(alpha-oléfines) comme l'isododécane ou l'isohexadécane, les huiles végétales transestérifiées et les huiles fluorées ; les composés de formule RₐCOOR_{b} dans laquelle Rₐ représente le reste d'un acide gras supérieur comportant de 5 à 29 atomes de carbone et R_{b} représente une chaîne hydrocarbonée contenant de 3 à 30 atomes de carbone.

20. Composition selon la revendication 19, **caractérisée en ce que** l'huile est choisie parmi l'huile d'avocat, l'huile de ricin, l'huile d'olive, l'isohexadécane, le polydécène , le myristate d'isopropyle, l'isononanoate d'isononyle, une paraffine liquide.

21. Composition selon l'une quelconque des revendications 2 à 20, **caractérisée en ce que** la ou le(s) huile(s) est ou sont présente(s) en une quantité comprise allant de 0,005 à 30 % en poids, de préférence de 0,01 à 15 % en poids par rapport au poids total de la composition.

22. Composition selon l'une quelconque des revendications 1 et 3 à 20, **caractérisée en ce que** la ou le(s) huile(s) est ou sont présente(s) en une quantité comprise allant de 0,01 à 3 % en poids, de préférence de 0,01 à 1 % en poids par rapport au poids total de la composition.

23. Composition selon l'une quelconque des revendications précédentes, **caractérisée en ce que** l'huile est pré-épaissie par le polymère comportant un hétéroatome.

24. Composition selon l'une quelconque des revendications précédentes, **caractérisée en ce que** le rapport pondéral huile(s)/polymère comportant un hétéroatome est supérieur ou égal à 50/50.

25. Composition selon la revendication 24, **caractérisée en ce que** le rapport pondéral huile(s)/polymère comportant un hétéroatome est supérieur ou égal à 60/40, de préférence compris entre 60/40 et 99/1.

26. Composition selon l'une quelconque des revendications précédentes, **caractérisée en ce que** l'huile présente une taille primaire moyenne en nombre comprise entre 1 et 100 µm, de préférence comprise entre 5 et 30 µm.

27. Composition selon l'une quelconque des revendications 1 et 3 à 26, **caractérisée en ce que** l'agent cationique est choisi parmi les polymères cationiques et les tensioactifs cationiques.

28. Composition selon la revendication 27, **caractérisée en ce que** le polymères cationique est choisi parmi les dérivés d'éther de cellulose quaternaires, les cyclopolymères cationiques, en particulier les homopolymères ou copolymères de chlorure de diméthyldiallylammonium, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium, et leurs mélanges.

29. Composition selon l'une quelconque des revendications 1 et 3 à 28, **caractérisée en ce que** le ou les polymères cationiques sont présents à des concentrations allant de 0,001 à 20%, de préférence de 0,05 à 10% et plus particulièrement de 0,1 à 5 % en poids par rapport au poids total de la composition.

30. Composition selon l'une quelconque des revendications précédentes, **caractérisée en ce que** les tensioactifs cationiques sont choisis parmi les sels d'amines grasses primaires, secondaires ou tertiaires, éventuellement polyoxyalkylénées, les sels d'ammonium quaternaire et leurs mélanges.

31. Composition selon la revendication 30, **caractérisée en ce que** les sels d'ammonium quaternaire sont choisis parmi :
- ceux qui présentent la formule générale (V) suivante : 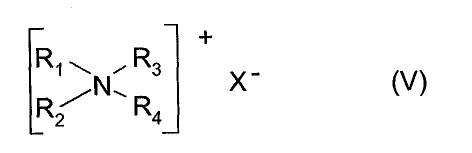 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline ;
- les sels de diammonium quaternaire de formule (VII) : 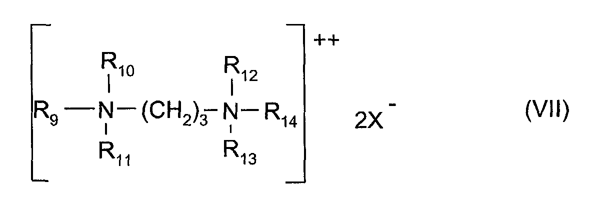 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X⁻ est un anion choisi dans le groupe des halogénures, acétates, phosphates, nitrates et méthylsulfates ;
- les sels d'ammonium quaternaire contenant au moins une fonction ester.

32. Composition selon la revendication 31, **caractérisée en ce que** les sels d'ammonium quaternaire de l'imidazoline sont choisis parmi ceux de formule (VI) suivante : 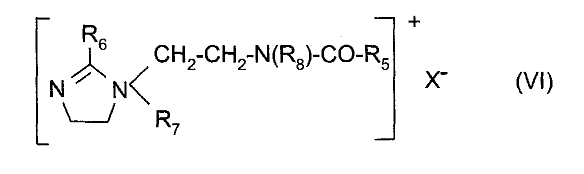 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène.

33. Composition selon l'une quelconque des revendications précédentes, **caractérisée en ce que** les tensioactifs cationiques sont choisis parmi le chlorure de béhényltriméthylammonium, le chlorure de cétyltriméthylammonium, le Quaternium-83, le chlorure de béhénylamidopropyl 2,3-dihydroxypropyl diméthyl ammonium et le chlorure de palmitylamidopropyltriméthylammonium.

34. Composition selon l'une quelconque des revendications précédentes, **caractérisée en ce que** les tensioactifs cationiques sont contenus en une quantité allant de 0,05 à 10 % en poids, de préférence de 0,1 à 8 % en poids par rapport au poids total de la composition.

35. Composition selon l'une quelconque des revendications précédentes, **caractérisée en ce qu'**elle comprend en outre au moins un agent conditionneur additionnel.

36. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

37. Composition selon la revendication 36, **caractérisée en ce que** le solvant cosmétiquement acceptable est choisi parmi les alcools inférieur en C₁-C₄ tel que l'éthanol, l'isopropanol, le tertio-butanol, le n-butanol ; les alkylèneglycols comme le propylèneglycol, les éthers de polyol ; les alcanes en C₅-C₁₀ et leurs mélanges.

38. Composition selon l'une quelconque des revendications précédentes, **caractérisée en ce qu'**elle comprend en outre des additifs tels que des polymères anioniques, non ioniques ou amphotères, des épaississants comme des acides ou des électrolytes, des opacifiants, des agents nacrants, des vitamines, des provitamines telles que le panthénol, des cires telles que les cires végétales, des céramides naturels ou synthétiques, des parfums, des colorants, des particules organiques ou minérales, des conservateurs, des agents de stabilisation du pH.

39. Composition selon l'une quelconque des revendications précédentes, **caractérisée par le fait qu'**elle se présente sous forme d'après-shampooing à rincer ou non, de compositions pour permanente, défrisage, coloration ou décoloration, ou encore sous forme de compositions éventuellement à rincer, à appliquer avant ou après une coloration, une décoloration, une permanente ou un défrisage ou encore entre les deux étapes d'une permanente ou d'un défrisage.

40. Composition selon l'une quelconque des revendications précédentes, **caractérisée en ce qu'**elle est se présente sous la forme d'un après-shampoing à rincer.

41. Procédé de traitement des matières kératiniques, telles que les cheveux, **caractérisé en ce qu'**il consiste à appliquer sur lesdites matières une quantité efficace d'une composition cosmétique selon l'une des revendications 1 à 40, puis à effectuer éventuellement un rinçage.
